# EUROPÄISCHE PATENTSCHRIFT

(11) **EP 0 763 037 B1**
(45) Veröffentlichungstag und Bekanntmachung des Hinweises auf die Patenterteilung: **14.11.2001**
(21) Anmeldenummer: 95921801.7
(22) Anmeldetag: 31.05.1995
(51) Int. Cl.: C07D 487/04, C07D 517/04, A61K 31/40, A61K 31/425, C07D 498/04, C07D 471/04

(54) **[a]-ANNELIERTE PYRROLDERIVATE UND DEREN ANWENDUNG IN DER PHARMAZIE**
[a]-ANNELATED PYRROLE DERIVATIVES AND PHARMACEUTICAL USE THEREOF
DERIVES DE PYRROLE [a]-ANNELES ET LEUR UTILISATION DANS LE DOMAINE PHARMACEUTIQUE

(30) Priorität: 01.06.1994 DE 4419247
(43) Veröffentlichungstag der Anmeldung: 19.03.1997
(73) Patentinhaber: MERCKLE GMBH, 89143 Blaubeuren (DE)
(72) Erfinder: LAUFER, Stefan, D-89143 Blaubeuren (DE); STRIEGEL, Hans, Günther, D-89143 Blaubeuren (DE); DANNHARDT, Gerd, D-55127 Mainz (DE)
(74) Vertreter: Kinzebach, Werner, Dr.
(86) Internationale Anmeldenummer: EP9502079
(87) Internationale Veröffentlichungsnummer: WO9532972

(56) Entgegenhaltungen:
- EP-A- 0 397 175
- ARCHIV DER PHARMAZIE, Bd. 327, Nr. 8, 1994 WEINHEIM DE, Seiten 509-514, G. DANNHARDT ET AL. 'C-5 Functionalized 6,7-diphenyl-2,3-dihydro-1H-pyrrolizines as inhibitors of bovine cyclooxygenase and 5-lipoxygenase'

## Beschreibung

Die Erfindung betrifft Pyrrole, die an Bindung a anneliert sind und deren Anwendung in der Pharmazie sowie pharmazeutische Mittel, welche diese Verbindungen enthalten.

Es ist bekannt, daß die Metabolisierung von Arachidonsäure auf zwei verschiedenen Wegen erfolgt. Beim Cyclooxygenase-Weg wird unter Einwirkung des Enzyms Cyclooxygenase die Arachidonsäure zu Prostaglandinen metabolisiert. Beim Lipoxygenase-Weg wird die Arachidonsäure unter Einwirkung von Lipoxygenasen zu den sogenannten Leukotrienen metabolisiert.

Die Prostaglandine sind an der Entstehung von Entzündung, Fieber und Schmerz beteiligt, während die Leukotriene bei der Entstehung von Asthma, Entzündungen und Allergien eine wichtige Rolle spielen. Zur Bekämpfung dieser Symptome werden häufig nicht-steroidale Antiphlogistika, wie Arylessigsäure-, 2-Arylpropionsäure- und Anthranilsäure-Derivate, eingesetzt. Diese Derivate hemmen die Cyclooxygenase und verhindern somit die Bildung der Prostaglandine aus Arachidonsäure. Die Anwendung derartiger Derivate ist jedoch aufgrund ihrer Nebenwirkungen nicht unbedenklich. Arzneimittel, welche die Lipoxygenase hemmen, sind im Handel jedoch nicht erhältlich.

Die EP-A-397 175 beschreibt Pyrrolizinverbindungen der Formel: 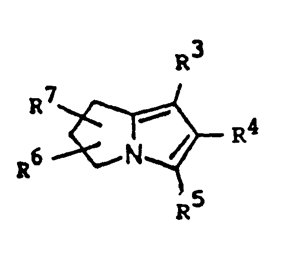 worin zwei der Reste R³, R⁴ und R⁵ unabhängig voneinander für H, C₅-C₈-Cycloalkyl, C₁-C₁₂-Alkyl oder Aryl, das gegebenenfalls durch ein oder zwei Reste substituiert ist, die ausgewählt sind unter Halogen, NO₂, C₁-C₄-Alkoxy, Hydroxy, C₁-C₄-Alkyl oder Phenoxy, stehen und der dritte der Reste R³, R⁴ und R⁵ für CHO, CO₂HO, COSC₁-C₄-Alkyl oder A - X steht, wobei A eine geradkettige oder verzweigte C₁-C₈-Alkylengruppe oder eine C₂-C₈-Alkenylengruppe bedeutet und X für CO₂H, SO₃H, CHO, OH oder SH steht. Diese Verbindungen sind Cyclooxygenase- und/oder Lipoxygenasehemmer und daher bei der Behandlung von Erkrankungen des rheumatischen Formenkreises und zur Prävention von allergisch induzierten Erkrankungen brauchbar.

Besonders wirksam sind diejenigen Verbindungen der obigen Formel, in der A-X für CH₂CO₂H steht. Diese besitzen allerdings eine hohe Neigung zur Decarboxylierung. Beispielsweise beträgt die Halbwertszeit in Chloroform ca. 1-2 h, während eine 1% alkalische, wäßrige Lösung mit ca. 2 %/Tag decarboxyliert.

Überraschenderweise wurde nun gefunden, daß bestimmte heterocyclische Verbindungen bei vergleichbarer Wirkung chemisch erheblich stabiler sind. Außerdem besitzen die Verbindungen auch neue Wirkqualitäten, wie Blutdrucksenkung, Lipidsenkung und Trachealrelaxation.

Gegenstand der Erfindung sind daher heterocyclische Verbindungen der Formel I: 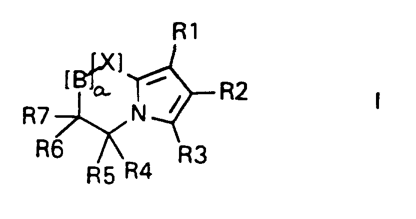 worin
zwei der Reste R¹, R² und R³, die gleich oder verschieden sein können, für ein Wasserstoffatom, einen Phenyl- oder Naphthylrest, der gegebenenfalls einen oder zwei Substituenten aufweist, die unter Halogen, Pseudohalogen, CF₃, NO₂, OH, C₁-C₈-Alkoxy, OCF₃, C₁-C₈-Alkyl oder Phenoxy ausgewählt sind, oder einen aromatischen heterocyclischen Rest, der ausgewählt ist unter einem Thiophen-, Thiazol-, Thiadiazol-, Furan-, Pyridin-, Pyrimidin-, Benzofuran- oder Chinolinrest und der gegebenenfalls durch Halogen, CF₃, C₁-C₈-Alkyl oder C₁-C₈-Alkoxy substituiert ist, stehen und
der dritte der Reste R¹, R² und R³ für A - Y steht,
- A: für C₁-C₈-Alkylen oder C₂-C₈-Alkenylen steht,
- Y: für CONR⁸R⁹ steht,

- R⁸: für H oder C₁-C₈-Alkyl steht,
- R⁹: für SO₂C₁-C₈-Alkyl, das gegebenenfalls durch Halogen substituiert ist,
oder
SO₂-Phenyl, das gegebenenfalls durch C₁-C₈-Alkyl substituiert ist, steht,
R⁴, R⁵, R⁶ und R⁷, die gleich oder verschieden sein können, für H oder C₁-C₆-Alkyl stehen oder zwei der vicinalen Reste für eine chemische Bindung zwischen den beiden Ringatomen, an die sie gebunden sind, stehen und die beiden anderen die angegebenen Bedeutungen besitzen,
- X: für CH₂, O oder S steht,
- B: für CH₂ steht, und
- a: für 0, 1 oder 2 steht, und
die optischen Isomere, pharmazeutisch verträglichen Salze und physiologisch leicht hydrolysierbaren Ester davon.

Die pharmazeutisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Milchsäure, Citronensäure, Äpfelsäure, Mandelsäure, Ascorbinsäure, Maleinsäure, Fumarsäure, Gluconsäure und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Zu den physiologisch leicht hydrolysierbaren Estern zählen insbesondere Alkyl-, Pivaloyloxymethyl-, Acetoxymethyl-, Phthalidyl-, Indanyl- und Methoxymethylester.

Der Ausdruck "Alkyl, Alkoxy etc." umfaßt geradkettige oder verzweigte Alkylgruppen, wie Methyl, Ethyl, n- und i-Propyl, n-, i- oder t-Butyl, n-Pentyl, Neopentyl, n-Hexyl etc. Soweit nicht anders angegeben, steht C₁-C₈-Alkyl insbesondere für C₁-C₆-Alkyl und besonders bevorzugt für C₁-C₄-Alkyl.

Der Ausdruck "Halogenatom" umfaßt ein Fluor-, Chlor-, Brom- oder Jodatom und insbesondere ein Fluor- oder Chloratom. "Pseudohalogen" steht insbesondere für CN, OCN, SCN oder N₃.

"Alkylen" oder "Alkenylen" steht für geradkettige oder verzweigte Alkylen- oder Alkenylengruppen mit vorzugsweise 1 bis 6 bzw. 2 bis 6 und insbesondere 1 bis 4 bzw. 2 bis 4 Kohlenstoffatomen. Die Alkylengruppe und insbesondere die Methylengruppe ist bevorzugt.

Bei dem "aromatischen heterocyclischen Rest" handelt es sich um einen 5- und 6-gliedrigen heterocyclischen Rest, der wie oben angegeben substituiert und kondensiert sein kann, und zwar um einen Thiophen-, Thiazol-, Thiadiazol-, Furan-, Pyridin-, Pyrimidin-, Benzofuran- oder Chinolinrest. Wenn der Heterozyklus substituiert ist, sind 1, 2 oder 3 Substituenten vorhanden, die ausgewählt sind unter Halogen, CF₃, C₁-C₈-Alkyl und C₁-C₈-Alkoxy. Bevorzugt ist ein Thiophen- oder halogen-, insbesondere chlorsubstituierter Thiophenrest, ein Furan-, Pyridin-, Benzofuran- oder Chinolinrest.

Wenn einer der Reste R¹, R² und R³ einen heterocyclischen Rest oder einen substituierten Phenyl- oder Naphthylrest bedeutet, dann steht vorzugsweise R² für einen derartigen Rest.

Die Substituenten der Phenyl oder Naphthylgruppe sind vorzugsweise ausgewählt unter Halogen, insbesondere Fluor oder Chlor, CF₃, NO₂ und Phenoxy. Wenn es sich bei der Phenyl oder Naphthylgruppe um einen Phenylring handelt, befinden sich die Substituenten vorzugsweise in mund/oder p-Stellung.

R⁹ steht insbesonder für SO₂CH₃, SO₂CF₃, SO₂Phenyl oder SO₂Toluyl.

Vorzugsweise steht der dritte der Reste R¹, R² und R³ in 5-Position des Pyrrolizidingerüstes.

Eine bevorzugte Ausführungsform sind die Verbindungen der obigen Formel I, worin zwei der Reste R¹, R² und R³ unabhängig voneinander für H, Phenyl, halogen- oder CF₃-substituiertes Phenyl (ein oder zwei Halogenatome) oder einen 5- oder 6-gliedrigen heterocyclischen Ring der oben definierten Art stehen.

Eine weiterhin bevorzugte Ausführungsform sind die Verbindungen der obigen Formel I, worin R¹ für H oder Phenyl steht, R² für Phenyl, halogen-substituiertes Phenyl oder einen 5- oder 6-gliedrigen heterocyclischen Ring steht und R³ für A - Y steht, wobei A und Y die oben angegebenen Bedeutungen besitzen.

Besonders bevorzugt steht A - Y für CH₂CONHSO₂R, wobei R für CH₃, CF₃, Phenyl oder Tolyl steht.

X steht vorzugsweise für CH₂; a steht vorzugsweise für O.

Eine besondere Ausführungsform sind die Verbindungen der obigen Formel I, worin zwei der Reste R⁴ und R⁶ bzw. R⁵ und R⁷ zusammen für eine chemische Bindung oder worin die Reste R⁴ bis R⁷ für H oder C₁-C₈-Alkyl stehen. Diese Verbindungen besitzen die Formel:

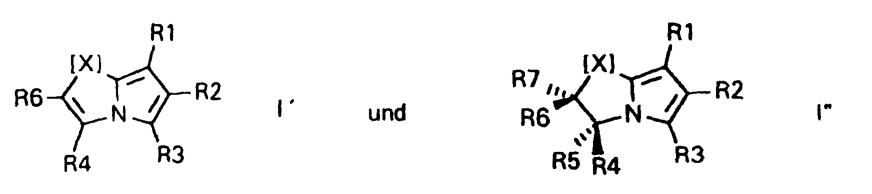

Die Reste R¹ bis R⁷ und X besitzen dabei die oben angegebenen Bedeutungen.

Eine weitere Ausführungsform sind die Verbindungen der Formel I" worin, falls X für CH₂ steht, R⁶ und R⁷ für C₁-C₈-Alkyl und R⁴ und R⁵ für Wasserstoff stehen; und, falls X für S steht, R⁶ und R⁷ für H und R⁴ und/oder R⁵ für C₁-C₈-Alkyl stehen.

Soweit die erfindungsgemäßen Verbindungen Aysmmetriezentren aufweisen, sind Racemate sowie optische Isomere (Enantiomere, Diastereomere) umfaßt.

Die Synthese der erfindungsgemäßen Verbindungen erfolgt analog zu den in den Fig. 1a-c, 2, 3a, 3b, 4, 5a und 5b beschriebenen Verfahren. Diese Verfahren sind zum Teil in der EP-A-397 157 beschrieben; auf diese Publikation einschließlich der darin erwähnten Literaturzitate wird hiermit Bezug genommen.

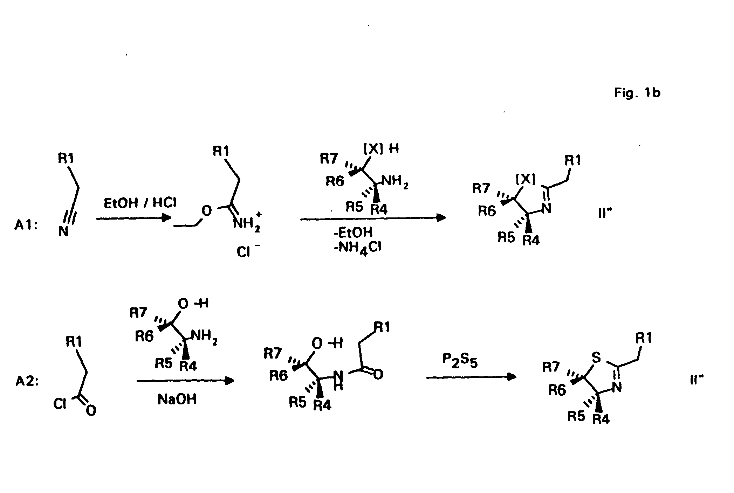

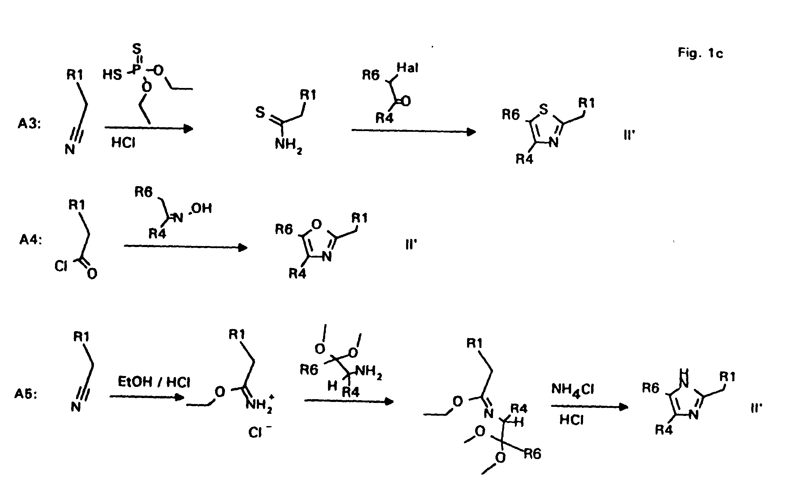

Ausgangsverbindungen für die Herstellung der erfindungsgemäßen Verbindungen sind die Verbindungen der Formel II: 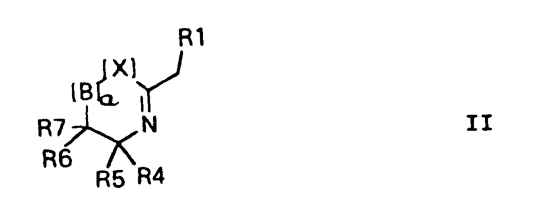 worin R¹, R⁴ bis R⁷ und X die oben angegebenen Bedeutungen besitzen. Diese Verbindungen sind literaturbekannt oder sie werden analog zu bekannten Verfahren hergestellt, z.B. durch die in EP -A-397 175 beschriebenen (X = CH₂) oder durch Umsetzung der von D- und L-Aminosäuren abgeleiteten Aminoalkohole, von Aminothiolen und von Diaminen mit den Imidestern entsprechend substituierter Carbonsäuren (Fig. 1b A1/A2). Die Verbindungen der Formel II werden mit den entsprechenden Verbindungen der Formel III 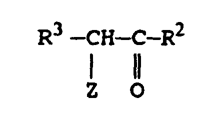 worin Z für Cl oder Br steht und R² und R³ die gewünschten Bedeutungen besitzen, umgesetzt, siehe Verfahren A. Die Verbindungen der Formel III sind ebenfalls literaturbekannt oder sie werden analog zu bekannten Verfahren hergestellt, beispielsweise werden diejenigen Verbindungen, bei denen R² für einen aromatischen heterocyclischen Rest steht, analog zu den von J.J. Riehl in C.R. Hebd. Seance. Acad. Sci. Ser.C (1957), 245, Seiten 1321-1322 beschriebenen Verfahren hergestellt. Die Umsetzung erfolgt in einem inerten Lösungsmittel (z.B. Ethanol, Methylenchlorid, Diethylether, Tetrahydrofuran) in Gegenwart einer geeigneten Base (z.B. NaHCO₃, Triethylamin). Wenn X für O oder S steht, erfolgt die Umsetzung vorzugsweise in einem Ether oder aromatischen Kohlenwasserstoff, z.B.

Diethylether, Benzol oder Toluol, wobei das quaternisierte Zwischenprodukt ausfällt. Dieses wird isoliert und in einem chlorierten Lösungsmittel, z.B. CH₂Cl₂, gelöst und mit einer Base, z.B. Triethylamin, behandelt.

Man erhält aus dieser Umsetzung die Verbindungen der Formel Ia:

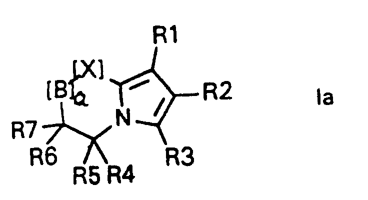

Wenn dabei mindestens einer der Reste R¹, R² und R³ für ein Wasserstoffatom steht, erhält man die Verbindungen der Formeln IVa bis IVc:

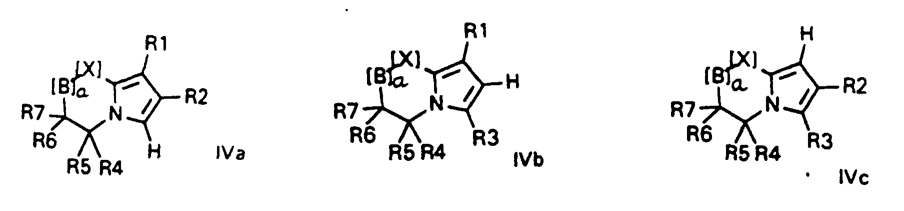

Je nach Stellung des Wasserstoffatoms leiten sich hiervon die Verbindungen der Reihe a, b oder c ab.

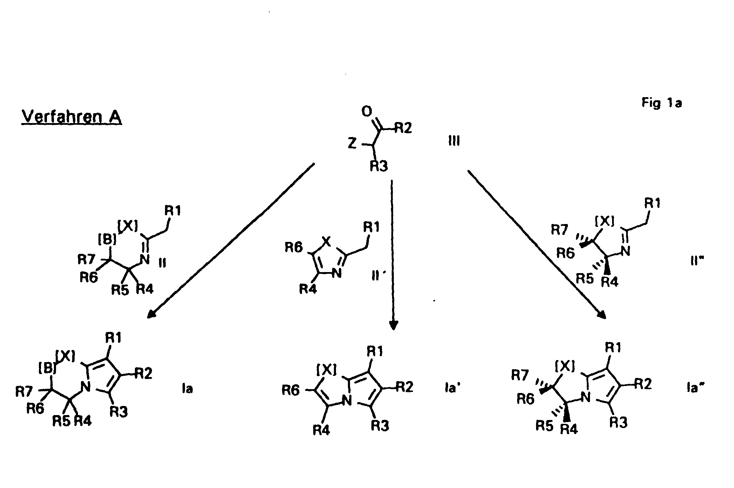

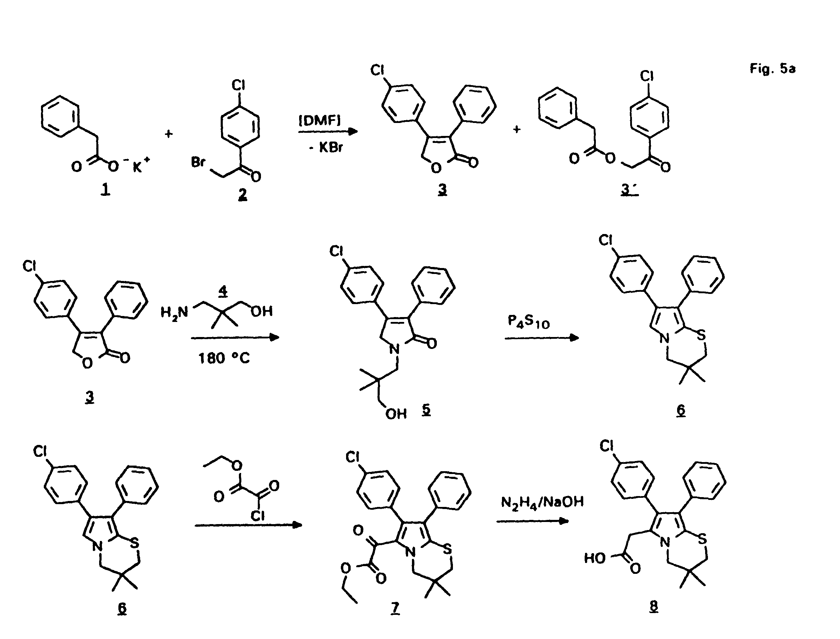

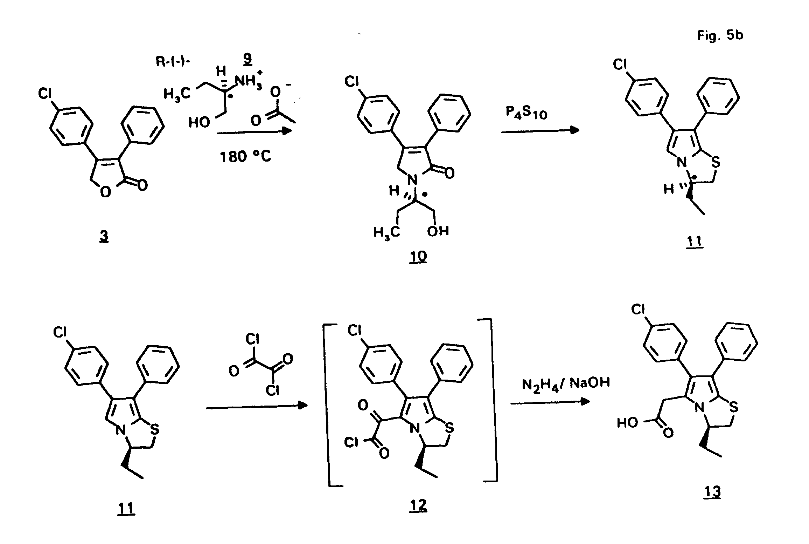

Diese Reaktion sowie die nachfolgend erwähnten Reaktionen sind in den Fig. 1a - c, 2, 3a, 3b, 4, 5a und 5b am Beispiel der Verbindungen der Reihe a skizziert. Analoges gilt für die Synthese und Derivatbildung der Verbindungen der Reihen b und c.

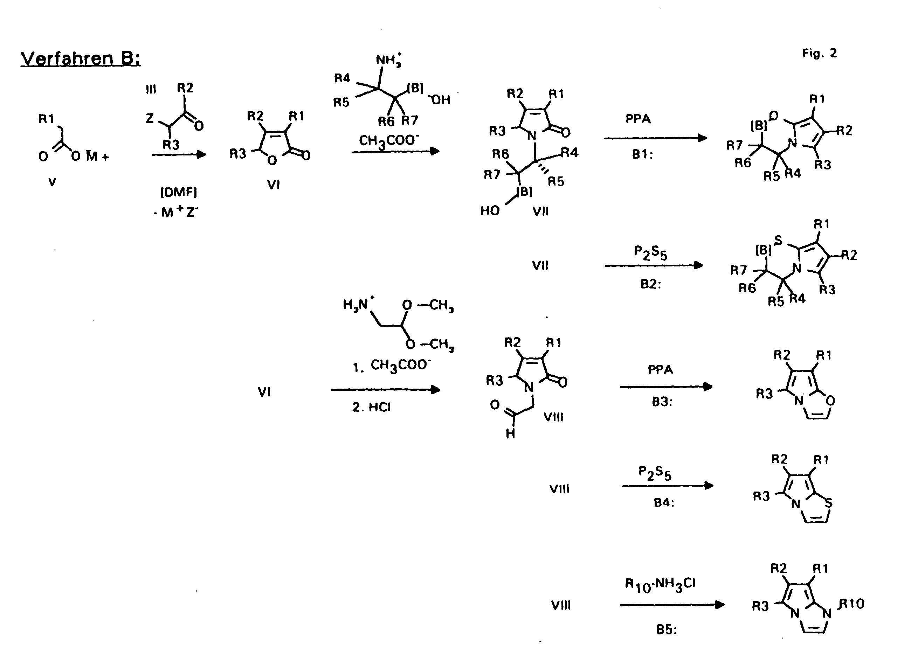

Zusätzlich zu dem in EP A-397 175 beschriebenen Verfahren (Verfahren A) findet zum Aufbau der Heterocyclen IVa, IVb und IVc mit X = O oder S ein weiteres Verfahren (Verfahren B) Anwendung (Fig. 2): Ausgangspunkt dieses Verfahrens sind entsprechend substituierte 2-(5H)Furanone (VI), die aus Carbonsäuresalzen der Struktur V und den Halogenaldehyden und -ketonen der Struktur III hergestellt werden (Fig. 2), analog zu den in der Literatur beschriebenen Methoden (a: Rio, G. und Sekiz, B. Bull. Soc. Chim. Fr. **1976**, 1491, 1495. b: Padwa, A. , Brookhart, T., Dehm, D. und Wubbels, G., J. Am. Chem. Soc. **1978**, *100,* 8247, 8259). Analog zu literaturbekannten Methoden werden diese in 1,5-Dihydro-2-pyrrolone (VII bzw. VIII) umgewandelt (c: Matsuda et al. Yakugaku Zasshi 95,[**1975**] 190, 194 ( C.A. 83 [**1975**] 42 780; d: Rio, G. und Sekiz, B., s.o.).

Die Cyclisierung zum annelierten Heterocyclus führt - abhängig vom verwendeten Kondensationsreagenz und von der zweiten funktionellen Gruppe des im vorhergehenden Schritt eingeführten bifunktionellen Amins NH₂-CR₄R₅CR₆R₇-[B]ₐ-OH bzw NH₂CH₂CH(OCH₃)₂ - zu teilhydrierten (Formel I", Fig. 2: B1 /B2) bzw. zu dehydrierten Formen (Formel I', Fig. 2: B3, B4, B5).

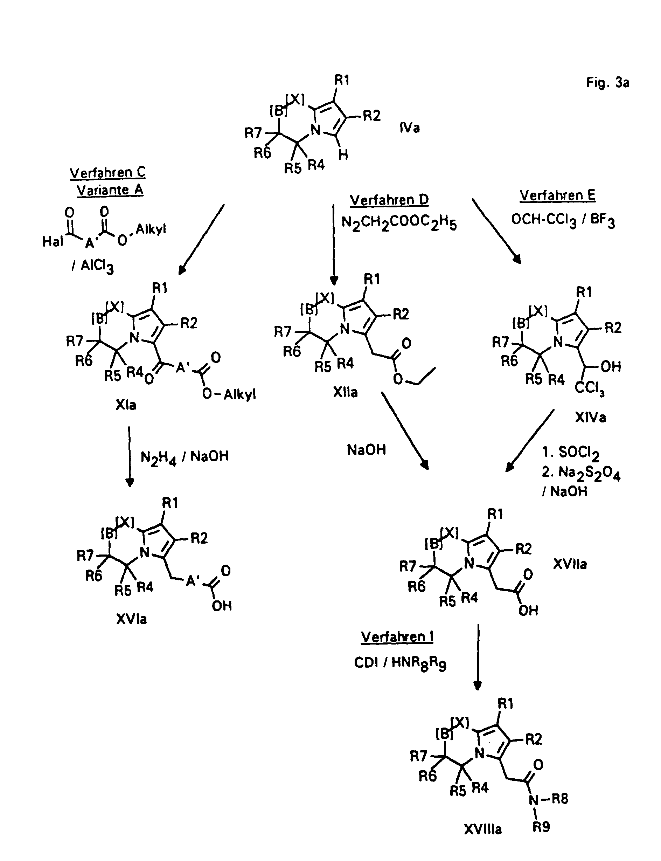

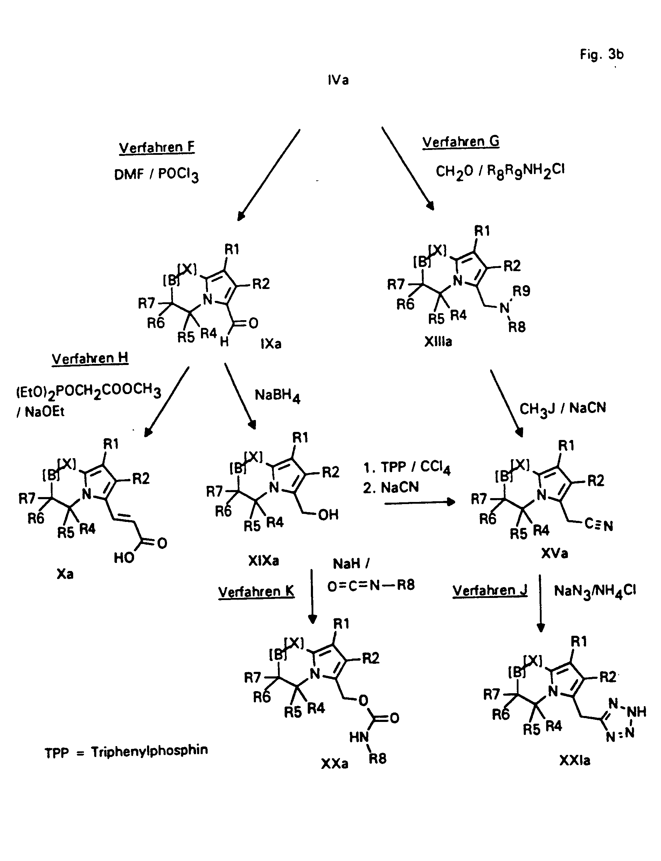

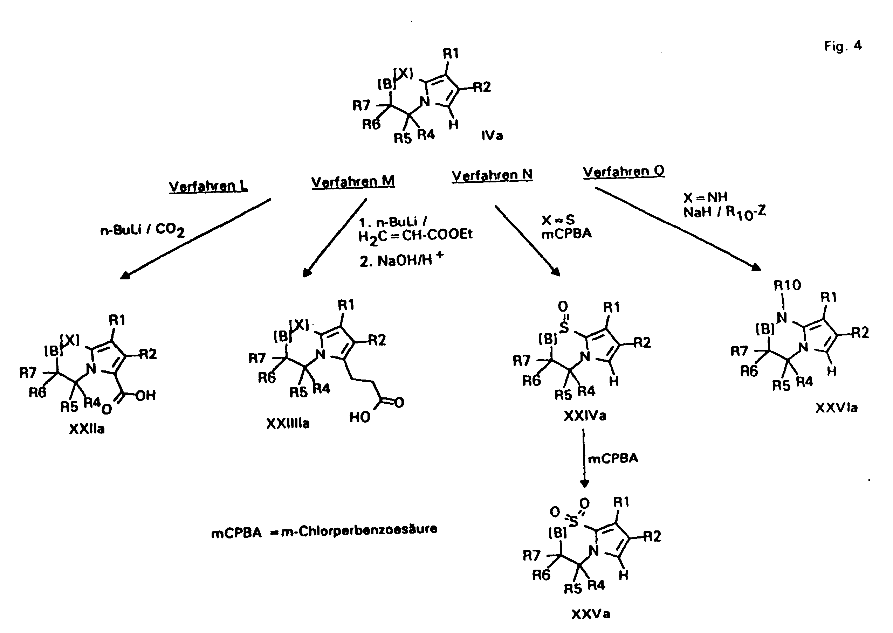

Zur Einführung des Substituenten A-CONR⁸R⁹ wird zunächst der Rest A-CO₂H nach Methoden eingeführt, die dem Fachmann bekannt sind. Zu diesen Methoden zählen beispielsweise:
a) Umsetzung einer Verbindung der Formel Ia mit einem Carbonsäurehalogenid HalOC-A'-COOAlkyl, worin A' für eine chemische Bindung, C₁-C₇-Alkylen oder C₂-C₇-Alkenylen steht und Hal für Cl oder Br steht (Fig. 3a, Verfahren C/Variante A). Die erhaltene Verbindung der Formel Ia, worin einer der Reste R¹, R² und R³ für CO-A'-CO₂Alkyl steht, wird dann mit einem Reagens behandelt, das zur Reduktion der Carbonylgruppe zu einer CH₂-Gruppe geeignet ist, beispielsweise Hydrazin, NaCNBH₃ oder Zink-Amalgam. Die Umsetzung mit dem Carbonsäurehalogenid wird in einem inerten Lösungsmittel, z.B. Diethylether oder Tetrahydrofuran, gegebenenfalls in Gegenwart eines Katalysators durchgeführt. Bevorzugt ist die Reduktion mit Hydrazin unter den Bedingungen einer Wolff-Kishner-Reduktion und insbesondere der Huang-Minlon-Variante davon. Die Reduktion mit Hydrazin erfolgt bevorzugt in einem hochsiedenden Alkohol, z.B. Diethylenglykol. Man erhält auf diese Weise die Verbindung der Formel XVI.
b) Zur Einführung der besonders bevorzugten Gruppe CH₂CO₂H stehen mehrere Methoden zur Verfügung (siehe Fig. 3a, 3b und 4). Die erste Möglichkeit besteht darin, daß man eine Verbindung der Formel IV mit Oxalylchlorid umsetzt (Fig. 5b), wobei man eine Verbindung der Formel I erhält, in der einer der Reste R¹, R² und R³ für COCO₂H steht. Diese Verbindung wird dann mit einem Reagens behandelt, das zur Reduktion der Ketocarbonylgruppe geeignet ist, beispielsweise Hydrazin, NaCNBH₃ oder Zink-Amalgam (vgl. auch Punkt a) oben).
   Eine weitere Möglichkeit besteht darin, eine Verbindung der Formel IVa mit einem Diazoessigsäurealkylester zu einer Verbindung der Formel Ic umzusetzen, in der einer der Reste R¹, R² und R³ für CH₂COOAlkyl steht. Diese Verbindung wird dann einer Esterspaltung zu der entsprechenden freien Carbonsäure unterworfen. (Fig. 3a, XVII).
   Die Umsetzung mit dem Diazoessigester erfolgt in einem inerten Lösungsmittel, beispielsweise Toluol oder Xylol in Anwesenheit von Kupferpulver oder komplexen Kupfer-I-salzen oder Kupfer-II-salzen. Die Reaktion wird bei erhöhter Temperatur durchgeführt, zweckmäßigerweise bei der Siedetemperatur des verwendeten Lösungsmittels.
   Eine weitere Möglichkeit besteht in der Umsetzung einer Verbindung der Formel IV mit Chloral zu einer Verbindung der Formel XIV und Behandlung der aktivierten Verbindung mit einem Dithionit, beispielsweise Natriumdithionit oder Rongalit (Natriumsalz der Hydroxymethansulfinsäure), siehe Fig. 3, Verfahren E.
c) Die Einführung einer Formylgruppe in den Pyrrolring erfolgt durch Umsetzung einer Verbindung der Formel IV mit Phosphoroxychlorid/Dimethylformamid (siehe Fig. 3b). Die Umsetzung wird in einem inerten Lösungsmittel, beispielsweise Benzol, Toluol oder Xylol, bei erhöhter Temperatur, zweckmäßigerweise am Siedepunkt des verwendeten Lösungsmittels, durchgeführt. Man erhält eine Verbindung der Formel IX, worin einer der Reste R¹, R² und R³ für CHO steht. Die Gruppe -CHO kann dann in üblicher Weise, z.B. mit LiAlH₄, in einem inerten Lösungsmittel, z.B. Tetrahydrofuran, zu der entsprechenden Hydroxymethylverbindung XIX reduziert werden (Fig. 3b). Diese kann dann für weitere Umsetzungen zur Einführung der gewünschten Gruppen dienen (Verfahren K,J, Fig. 3b).
   Die Formylgruppe kann in einer unter üblichen Bedingungen durchgeführten Wittig-Reaktion in eine entsprechende Alkenylengruppe unter Bildung der Verbindung X überführt werden (siehe Verbindung X in Fig. 3b). Diese wiederum kann gewünschtenfalls in üblicher Weise zu der entsprechenden Alkylenverbindung (XXIII, Fig. 4) hydriert werden.
d) Umsetzung einer Verbindung der Formel IV mit einem Anhydrid der Formel: 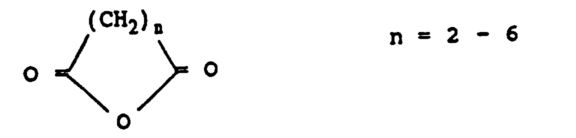 ergibt die entsprechenden Ketocarbonsäuren der Formel I, worin einer der Reste R¹, R² und R³ für CO(CH₂)ₙCO₂H steht. Die Ketocarbonylgruppe kann mit dem bereits erwähnten Reagens zu einer CH₂-Gruppe reduziert werden (siehe Fig. 3a, XI-XVI).

Die Herstellung der Verbindungen der Formel I, worin einer der Reste R¹, R² und R³ für A-CONR⁸R⁹ steht, erfolgt dann ausgehend von dem entsprechenden aktivierten Derivat der Carbonsäure der Formel Ia, worin einer der Reste R¹, R² oder R³ für ACO₂H steht, durch Umsetzung mit dem entsprechenden Sulfonamid, Amin, Amid oder Hydroxylamin (s. Fig. 2). Geeignete aktivierte Carbonsäurederivate sind dem Fachmann bekannt, bevorzugt ist das Imidazolidderivat.

Die Umsetzung wird in einem inerten Lösungsmittel, beispielsweise einem Ether, wie Diethylether oder Tetrahydrofuran, in Gegenwart einer Base, beispielsweise Natriumhydrid, durchgeführt. Die Reaktionstemperatur liegt im Bereich, der von Raumtemperatur bis zum Siedepunkt des Lösungsmittels reicht. Zweckmäßigerweise wird die Umsetzung bei Raumtemperatur durchgeführt.

Die erfindungsgemäßen Verbindungen haben sich als potente und chemisch stabile Cyclooxygenase- und/oder Lipoxygenasehemmer erwiesen. Sie sind daher bei der Behandlung von Erkrankungen brauchbar, die mit einer Veränderung der Arachidonsäuremetabolisierung einhergehen. Insbesondere sind zu nennen Erkrankungen des rheumatischen Formenkreises und zur Prävention von allergisch induzierten Erkrankungen brauchbar. Die erfindungsgemäßen Verbindungen stellen somit wirksame Antiphlogistika, Analgetika, Antipyretika, Antiallergika und Broncholytika dar oder sind antibronchokonstriktorisch wirksam und daher zur Thromboseprophylaxe und zur Prophylaxe des anaphylaktischen und septischen Schocks sowie zur Behandlung dermatologischer Erkrankungen, wie Psoriasis, Urtikaria, akute und chronische Exantheme allergischer und nicht-allergischer Genese brauchbar. Außerdem sind sie brauchbar zur Behandlung des Fettstoffwechsels, insbesonders zur Senkung der Cholesterolwerte.

Die erfindungsgemäßen Verbindungen besitzen im Vergleich zu den entsprechenden Carbonsäuren eine erheblich größere Stabilität in Lösung. So decarboxyliert 6-(4-Chlorphenyl)-2,2-dimethyl-7-phenyl-2,3-dihydro-1H-pyrrolizin-5-yl-essigsäure in Chloroform mit einer Halbwertszeit von ca. 1 h und in wäßriger Lösung (Na-Salz) mit ca. 2 % / Tag. Das analoge Methansulfonimid hingegen ist bei weitgehend erhaltenem Wirkprofil stabil.

Die erfindungsgemäßen Verbindungen können entweder als einzelne therapeutische Wirkstoffe oder als Mischungen mit anderen therapeutischen Wirkstoffen verabreicht werden: Sie können als solche verabreicht werden, im allgemeinen werden sie jedoch in Form pharmazeutischer Mittel verabreicht, das heißt, als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wäßriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Das Wirkungsspektrum der Verbindungen wurde anhand folgender Tests untersucht:
1) Phenylchinon-Writhing-Test an der Maus p.o., S. Irwin, Psychopharmacologia, 13:222-257, 1968;
2) Formalin-Analgesic-Test an der Maus p.o., B. Rubin et al., Endocrinol., 49:429-439, 1951;
3) Hemmung der Arachidonsäure-induzierten Plättchenaggregation, V. Bertele et al., Science 220:517-519 (1983) ;
4) Entzündungshemmung am Rattenpfotenödem, C.A. Winter et al., Proc. Exper. Biol. Med., 111:544-547 (1962);
5) Trachealrelaxierung am Meerschweinchen, F.P. Ludnena et al., Arch. Int. Pharmacodyn., 111:392-400, 1957;
6) Cholesterinsenkende Wirkung an der Maus, C.E. Day et al., Atherosclerosis Drug Discovery, Edit. Charles E. Day, Plenum Publishing Corp., New York, 1976, 231-249.
7) IC₅₀LO/CO: Hemmwirkung auf die Enzyme Cyclooxygenase (CO) und 5-Lipoxygenase (LO), Dannhardt et al., J. Pharm. Pharmacol. 1992, 44: 419-424.

Die Ergebnisse sind der nachfolgenden Tabelle 1 angegeben:

**Tabelle 1:**

| Biologische Daten: | | | | | | | |
|---|---|---|---|---|---|---|---|
| **Verbindung**^{**1)**} | 1 | 2 | 3 | 4 | 5 | 6 | 7 |
| **3** | x | x | x | - | x | x | - |
| **4** | x | - | x | x | x | - | 2,3/1,5x10⁻⁷ |
| **5** | - | - | x | - | - | x | - |
| **6** | - | - | x | - | - | - | - |
| **7** | - | - | x | x | x | - | - |
| **8** | - | - | x | - | - | x | - |
| **9** | x | x | x | x | - | - | - |
| **10** | x | - | x | - | - | x | - |
| **11** | x | - | x | - | - | - | - |
| **12** | - | x | x | - | - | x | - |
| **13** | - | - | - | - | x | x | - |

| | | | | | | | |
|---|---|---|---|---|---|---|---|
| ¹⁾ Verbindung des Beispiels Nr. | | | | | | | |

Die nachfolgenden Beispiele erläutern die Erfindung. Alle Temperaturangaben sind unkorrigiert. Die IR-Spektren wurden, soweit nicht anders angegeben, mit KBr-Preßlingen aufgenommen. Die NMR-Spektren sind, sofern nicht anders vermerkt, 200 MHz-Spektren, aufgenommen in CDCl₃ mit Tetramethylsilan (TMS) als internem Standard. Die IR-Spektren sind in cm⁻¹ und die NMR-Spektren in 6(ppm) angegeben.

### Beispiele

### Allgemeine Herstellungsvorschrift für arylsubstituierte [a]-bzw. [1,2]-annelierte Pyrrole (Pyrrolo[1,2-a]pyrrole = Pyrrolizine, Pyrrolo[1,2-a]pyridine = Indolizine, Pyrrolo[1,2-a]azepine)

Zu einer Lösung von 20 mmol ω-Bromacetylverbindungen in 100 ml Methylenchlorid werden 20 mmol des entsprechenden cyclischen Imin-Derivates in 50 ml Methylenchlorid rasch zugetropft und bei Raumtemperatur 4 Stunden unter Feuchtigkeitsausschluß gerührt. Anschließend gibt man 30 ml 5%ige wäßrige NaHCO₃-Lösung zu und rührt intensiv weitere 4 Stunden. Nach Zugabe von 200 ml Wasser wird die organische Phase abgetrennt, über Na₂SO₄ getrocknet und unter vermindertem Druck verdampft. Der Rückstand wird mit Methanol zur Kristallisation gebracht und gegebenenfalls aus Methanol umkristallisiert.

### Allgemeine Herstellungsvorschrift für arylsubstituierte [a]-bzw. [1,2]-annelierte Pyrrol-5-yl-oxoessigsäuren

Zu einer Lösung von 1,4 mmol Oxalsäureethylesterchlorid in 20 ml trockenem Methylenchlorid werden unter Rühren 1,3 mmol entsprechend substituiertes anneliertes Pyrrol, gelöst in 20 ml trockenem Methylenchlorid, unter Rühren zugetropft und 20 Minuten gerührt. Nach vorsichtigem Zusatz von 40 ml Wasser wird die organische Phase abgetrennt und über Na₂SO₄ getrocknet. Der nach Abzug des Lösungsmittels verbleibende Rückstand wird in 20 ml Diisopropylether suspendiert, abgesaugt und noch zwei mal mit je 5 ml Diisopropylether nachgewaschen.

### Allgemeine Herstellungsvorschrift für arylsubstituierte [a]-bzw. [1,2]-annelierte Pyrrol-5-yl-essigsäuren

2 mmol des entsprechenden Oxoesterderivates werden mit 2 ml Diethylenglykol und 1,5 ml 80%igem Hydrazinderivat versetzt und 30 Minuten bei 60°C gerührt. Anschließend werden 2,1 g Kaliumhydroxid zugesetzt und das Reaktionsgemisch wird unter Rühren zwei Stunden auf 140°C erhitzt.

Das noch warme Gemisch wird auf 20 ml Eiswasser gegeben und mit verdünnter Phosphorsäure auf pH = 3 - 4 gebracht, wobei sich das Rohprodukt fest abscheidet. Man saugt ab, wäscht mehrfach mit Wasser nach, trocknet im Vacuum und wäscht anschließend mehrfach mit wenig Diisopropylether.

### Allgemeine Herstellungsvorschrift für N-sulfonylierte [a]-bzw. [1,2]-annelierte Arylpyrrolcarbonsäureamide

### Mischung A:

2,6 mmol der betreffenden Pyrrolcarbonsäure werden mit 5 mmol Carbonyldiimidazol in 25 ml trockenem Tetrahydrofuran 1 h bei Raumtemperatur gerührt.

### Mischung B:

3 mmol des entsprechend substituierten Sulfonamids werden unter Argon-Atmosphäre in 20 ml trockenem Tetrahydrofuran gelöst, mit 3,3 mmol Natriumhydrid (Mineralölsuspension) versetzt und 1h bei Raumtemperatur gerührt.

Mischung B wird unter Argon-Atmosphäre zu Mischung A gegeben und 40 h gerührt. Die Suspension wird auf 40 ml Eiswasser gegossen, mit verdünnter Phosphorsäure auf pH = 4 gebracht und mit Diethylether mehrfach extrahiert. Nach Trocknen der organischen Phase über Na₂SO₄ und Abziehen des Lösungsmittels, wird der verbleibende Rückstand aus Isopropanol umkristallisiert.

Die erhaltenen Zwischenverbindungen und Endverbindungen sowie ihre physikalischen Daten sind in den nachfolgenden Tabellen 2 bis 5 angegeben. Die Zwischenverbindungen für die Beispiele 1, 2 und 13 sind in der EP-A-397 175 beschrieben, diejenigen für die Beispiele 3 bis 6, 9 und 10 werden analog hergestellt.

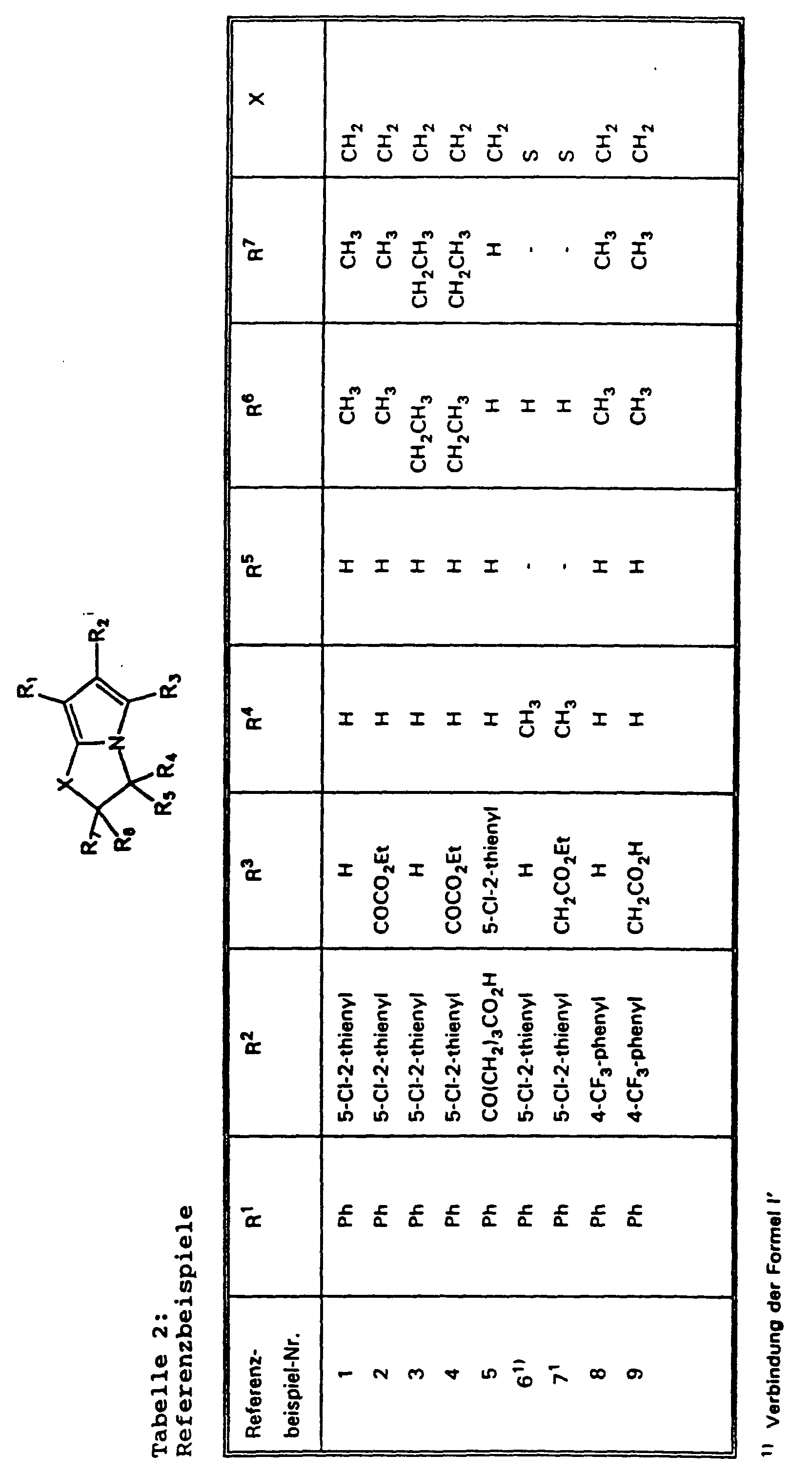

**Tabelle 3**

| Verbindung des Referenzbeispiels-Nr. | |
|---|---|
| 1 | Schmp.: Öl |
| | IR: 2950, 1656, 1596, 1444, 1414, 1382, 792, 759, 697 |
| | NMR: 7,29-7,17(m, 5H,arom); 6,71(s,1H,N-CH-); 6,70 (AB, J = 3,5, =CH-); 6,49 (AB,J=3,5,=CH-); 3,72(s,2H,-CH₂-N); 2,75(s,2H,-CH₂-); 1,27(s,6H,-CH₃) |
| 2 | Schmp.: 133,0°C |
| | IR: 2955, 1736, 1619, 1467, 1426, 1373, 1241, 1179, 1049, 701 |
| | NMR: 7,26-7,10(m, 5H, arom); 6,82 (AB. J=3,7, -CH=); 6,77 (AB, J=3,7, -CH=); 4,22(s,2H. -CH₂N-); 3,87(q, 2H, J = 7,0, ethyl); 2,82(s,2H, -CH₂-); 7,31(s,6H,-CH₂);1,19(t,3H,J=7,0,ethyl) |
| 3 | Schmp. 80,3°C |
| | IR: 2995, 1550, 1447, 1380, 1157, 1062, 986, 786, 758, 694 |
| | NMR: 7,30-7,15(m,5H,arom); 6,68(d,AB,1H, J=4,0, thienyl); 6,47 (d,AB, 1H, thienyl) (6,67(s,1H,-N-CH =); 3,74(s,2H,-CH₂-N); 2,73(s,2H,-CH₂pyr); |
| 4 | Schmp.: 126,8 |
| | IR: C = O; 1750, 1629 |
| | NMR: 7,31-7,09 (m, 5H, arom); 6,81 +6,76(AB,2H, J=3,7, -CH=CH-) 4,23(s,2H,-CH₂-N-); 3,87(q,2H,J=7,2, ethylester); 2,79 (s.2H. CH₂) |
| 5 | Schmp. 126°C |
| | IR: 1660, 1706, (C=O) |
| | NMR: 8,7-8,5(2H, Ar); 8,5-8,35(3H;Ar); 6,82(H_{A}, J_{AB}=3.76Hz); 6,76 (H_{B}, J_{AB} = 3,75 Hz); 5,08 (t,2H, CH₂); 4,14(t,2H, CH₂); 3,67(quint.. 2H, CH₂); 3,53(t,2H, CH₂); 3,33 (t,2H, CH₂); 2,95 (quint.. 2H, CH₂). |
| 6 | NMR: 7,24-7,06 (m, 5H, Ar); 7,16 (s, 1H); 6,80, 6,62 (AB, J = 3,8 Hz, Thiophen-H) ; 6,31 (9,1H, J = 0,8 Hz); 2,40 (d, 3H, J = 0,8 Hz) |
| 7 | NMR: 7,26-7,03 (m, 5H, Ar); 6,80, 6,62 (AB, J = 3,8 Hz, Thiophen-H); 6,30 (9, 1H, J = 0,8 Hz); 3,51 (s, 2H, CH2); 2,62 9,2H, 6,8 Hz); 2,38 (d, 3H, 0,8 Hz); 1,12 (t, 3H, J = 6,8 Hz) |
| 8 | Schmp.: 104 - 105°C |
| | JR: 3435, 2960, 1608, 1315, 1158, 1121, 843, 762, 698 |
| | NMR: 7,48-7,15 (m, 9H, Ar); 6,75 (s, 1H); 3,75 (s, 2H, CH₂-N); 2,79(s, 2H, -CH₂-); 1,28 (s, 6H, CH₃) |
| 9 | Schmp. 174 - 174,5°C |
| | IR: 3420, 2960, 1706, 1319, 1156, 1110, 846, 758, 695 |
| | NMR: 7,5 - 7,04 (m, 9H, Ar); 3,77 (s, 2H, CH₂CO₂H); 3,6 (s, 2H, CH₂-N); 2,86 (s, 2H, -CH₂); 1,30 (s. 6H, CH₃) |

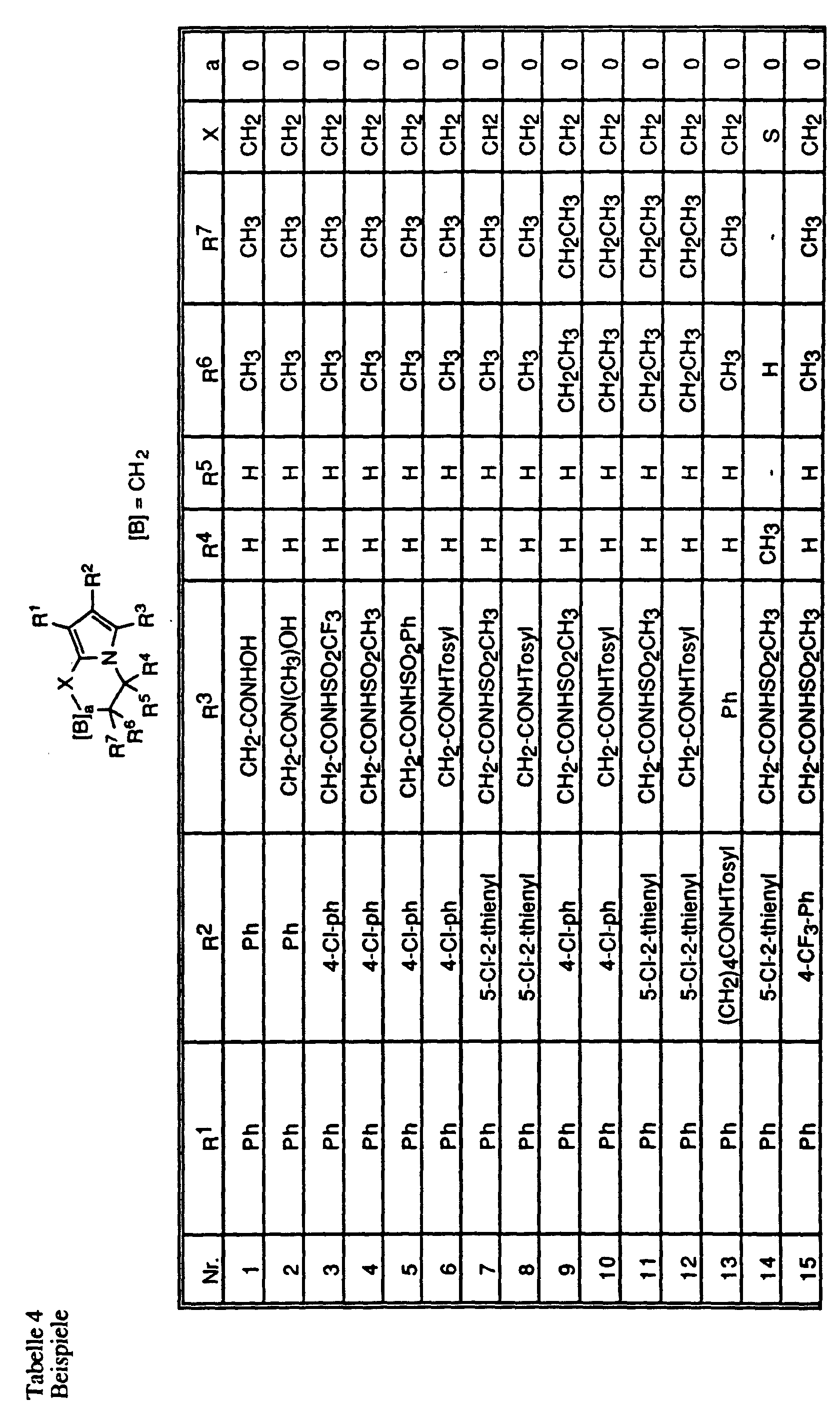

**Tabelle 5**

| Verbindung des Beispiels-Nr. | |
|---|---|
| 1 | Schmp.: 77,1 °C |
| | IR. 3255 (OH); 1651 (C = O) |
| | NMR: 7,12-6,67 (m, 10H, Ar.); 3,53 (s, 2H, CH₂-3); 3,37 (s, 2H, -CH₂-Py); 2,73 (s, 2H, CH₂-1); 1,38 (s, 6H, CH₃) |
| 2 | Schmp. 160,1 °C |
| | IR: 3450 (OH) 1620 (C = O) |
| | NMR: 7,30 - 6,90 (m, 10H, Ar.); 3,81 (s, 2H, CH₃-3); 3,68 (s, 2H, CH₂-Py); 2,93 (s, 3H, CH₃-N); 2,85 (s, 2H, CH₂-1); 1,3 (s, 6H, CH₃) |
| 3 | Schmp.: 178 - 179 °C |
| | IR: 3415 (-NH-), 1689 (-C=O). 1531, 1010 (SO₂) |
| | NMR: δ = 7,4 - 7,0 (m, 9H, 2 Ar); 5,42 (b; 1H. -NH); 3,72 (s, 2H, CH₂), 3,67 (s; 2H,-CH₂-); 2,87 (s, 2H, CH₂), 1,30 (s, 6H, 2CH₃) |
| 4 | Schmp.: 187 - 188 °C |
| | IR: 3240, 2955, 1729, 1480, 1449, 1397, 1325, 1180, 1102, 695, 3190, 2955, 1717, 1450, 1400, 1341, 1321, 1170, 1115, 974, 693, 500 |
| | NMR: δ(ppm): 7,91 (s, 1H, -NH-); 7,32-7,01 Im, 9H, arom); 3,69 (s, 2H, -CH₂-N-); 3,67 (s, 2H, CH₂-C=O); 3,10 (s, 3H, CH₃-s); 2,87 (s, 2H, -CH₂-); 1,31 (s, 6H, -CH₃) |
| 5 | Schmp. 196-197 °C |
| | IR: 3480 (-NH-) 1716 (-C=O), 1447 (-SO2-) cm⁻¹ 1175, 1131, 1084 (SO₂) |
| | NMR: δ = 8,3 (s. b NH), 7,9 - 6,9 (m, 14 H, 3M); 3,55 (s, 4H, 2-CH₂-); 2,83 (s. 2H, CH₂), 1,26 (s. 6H, 2 CH₃) |
| 6 | Schmp. 207 - 209 °C |
| | IR: 3295 (-NH-), 1721 (-C=N), 1413, 1183, 1084 (-SO₂) NMR: 7,8 - 7,7 (m, 2H, AA'), 7,35 - 6,95 (m, 11H, 2 Ar + BB'); 3,55 (5,2H, CH₂); 3,54 (s; 2H, CH₂); 2,84 (s, 2H; CH₂); 2,45 (s, 3H, Ar-CH₃); 1,26 (s; 6H; 2-CH₃) |
| 7 | Schmp.: 163 °C |
| | IR: 3220, 2950, 1721, 1432, 1395, 1341, 1176, 1113, 971, 878 |
| | NMR: 7,31-7,14 (m, 5H, arom.); 6,83+6,56(AB.2H.J=3,7Hz-CHCH-); 3,72(s,2H,); 3,69(s,2H); 3,44(s,3H,-SO₂CH₃); 2,84(s,2H, -CH₂-); 1,30(s,6H,-CH₃) |
| 8 | Schmp.: 188°C |
| | IR: 3235 (-NH-), 1725 (-C=O), 1442, 1166, 1083 (SO₂-) |
| | NMR: 8,1(b,1H,-NH), 7,9-7,8(m,2H,AA',Ar), 7,4-7,1(m,7H,Ar+BB') 6,74, 2,82(s,2H, CH₂); 2,29-2,41(m,2H,CH₂-COOH) (6,45(AB,JAB=3,75Hz); 3,58(s;2H,CH₂); 3,56(s,2H,CH₂) 2,80(s,2H,CH₂),2,45(s,3H,Ar-CH₃);1,25(5,6H;2CH₃) |
| 9 | Schmp. 191 °C |
| | IR: 3435, 3225, 1721, 1598, 1447, 1400, 1340, 1320, 1111, 971 |
| | NMR: 7,32-7,01 (m, 9H, arom); 3,71 (s, 2H, -CH₂-CO-7); 3,68 (s, 2H, ); 3,11 (s, 3H, -SO₂-CH₃); 2,85 (s, 2H, -CH₂-); 1,64 (q, 4H, J = 7,5, ethyl), 0,91 (t, 6H, J = 7,5, ethyl) |
| 10 | IR: 3225 (-NH-), 1721 (-C = O), 1439 (-SO₂-) cm⁻¹ 1184 ((SO₂), 1081 (SO₂) |
| | NMR: 7,79 - 6,95 (m, 13H, arom), 3,57 (s, 2H, -CH₂-C = O), 3,54 (s, 2H, -CH₂-N-), 2,8 (s, 2H,-CH₂-), 2,44 (s, 3H, Ph-CH₃) |
| 11 | Schmp.: 140-142°C |
| | IR: 3260 (-NH-), 1722(-C=O), 1437 (-SO₂-) 1327, 1113 |
| | NMR: 7,4-7,1 (m. 5H, Ar); 6,82/6,55 (AB-System, J_{AB} = 3,8 Hz 3,72 (s.2H, CH₂); 3,71(s,2H, CH₂); 3,21 (s; 3H, SO₂CH₃₎, 2,82(s,2H,CH₂), 1,619 (q,4H, 2-CH₂, J =7,4Hz; 0,899 (t, 6H, J=7,4Hz, 2CH₃-) |
| 12 | Schmp.: 158 - 1 60°C |
| | IR: 3225 (-NH-). 1721 (-C=O); 1432, 1184, 1084 (-SO₂₎ |
| | NMR: 8,02 (b,1H; NH-); 7,9-7,8 (m;2H; AA') 7,4-7,1(m= ,7H, Ar+BB'); 6,740/6,446(AB,2H. JAB=3,7Hz); 3,58(s,4H, 2-CH₂); 2,79 (s,2H,CH₂), 2,45(s;3H,Ar-CH₃); 1,57 (q,4H;2CH₂); 0,861 (t,6H,2CH₃) |
| 13 | Schmp. 210 °C unter Zersetzung |
| | IR: 3300 (NH); 1720 (C = O) |
| | NMR: 1,20 - 1,58 (m, 4H, -CH₂-CH₂-); 1,26 (s, 6H, CH₃); 1,99 - 2,20 (m, 2H, CH₂-CO); 2,42 (s, 3H, Ar-CH₃); 2,83 (s. 2H, CH3); 2,28 - 2,39 (m. 2H, CH₂-Py); 3,75 (s, 2H, CH₂), 6,94 - 7,71 (m, 14H, Ar.) |
| 14 | Schmp.: 203-204 °C |
| | IR: 3430, 3175, 3161, 1678, 1596, 1466, 1438, 1341, 1133, |
| | NMR: 8,2 -7,7 (b, 1H, NH), 7,4 - 7,1 (m, 5H, Ar.); 6,90 (d, 1H, J=4,0Hz, thien.), 6,69 (d, 1H. J=4,0 Hz, thien.); 6,38 (q, 1H. J=0,8Hz, thiaz.);4.07 (s, 2H, CH₂);3,271 (s, 3H, SO₂CH₃); 2,554 (d. 3H, J=0,8Hz). |
| 15 | Schmp. > 190 °C u.Z. |
| | IR: 3480 (CNH); 1722 (C = O) |
| | NMR: 7,51 - 7,08 (m, 9H, Ar); 3,67 (s, 2H, CH₂-C); 3,58 (s, 2H, CH₂-). 3,07 (s, 3H, CH₃); 2,86 (s, 2H, CH₂); 1,28 (s, 6H, CH₂) |

## Patentansprüche

1. Heterocyclische Verbindungen der Formel I: 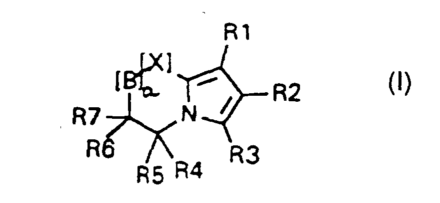 worin
zwei der Reste R¹, R² und R³, die gleich oder verschieden sein können, für ein Wasserstoffatom, einen Phenyl- oder Naphthylrest, der gegebenenfalls einen oder zwei Substituenten aufweist, die unter Halogen, Pseudohalogen, CF₃, NO₂, OH, C₁-C₈ -Alykoxy, OCF₃, C₁-C₈-Alkyl oder Phenoxy ausgewählt sind, oder einen aromatischen heterocyclischen Rest, der ausgewählt ist unter einem Thiophen-, Thiazol-, Thiadiazol-, Furan-, Pyridin-, Pyrimidin-, Benzofuran- oder Chinolinrest und der gegebenenfalls durch Halogen, CF₃, C₁-C₈-Alkyl oder C₁-C₈-Alkoxy substituiert ist, stehen und
der dritte der Reste R¹, R² und R³ für A - Y steht,
A für C₁-C₈-Alkylen oder C₂-C₈-Alkenylen steht,
Y für CONR⁸R⁹ steht,
R⁸ für H oder C₁-C₈-Alkyl steht,
R⁹ für SO₂C₁-C₈-Alkyl, das gegebenenfalls durch Halogen substituiert ist,
oder
SO₂-Phenyl, das gegebenenfalls durch C₁-C₈-Alkyl substituiert ist, steht,
R⁴, R⁵, R⁶ und R⁷, die gleich oder verschieden sein können, für H oder C₁-C₈-Alkyl stehen oder zwei der vicinalen Reste für eine chemische Bindung zwischen den beiden Ringatomen, an die sie gebunden sind, stehen und die beiden anderen die angegebenen Bedeutungen besitzen,
X für CH₂, O oder S steht,
B für CH₂ steht, und
a für 0, 1 oder 2 steht, und
die optischen Isomere, pharmazeutisch verträglichen Salze und physiologisch leicht hydrolysierbaren Ester davon.

2. Verbindungen nach Anspruch 1 der Formel I, worin zwei der Reste R¹, R² und R³ unabhängig voneinander für ein Wasserstoffatom, einen Phenylrest, einen durch ein oder zwei Halogenatome oder CF₃ substituierten Phenylrest oder einen wie in Anspruch 1 definierten, gegebenenfalls substituierten aromatischen, heterocyclischen Rest stehen, der dritte der Reste R¹, R² und R³ für A - Y steht, wobei
A für C₁-C₈-Alkylen steht und
Y die in Anspruch 1 angegebenen Bedeutungen besitzt.

3. Verbindungen nach Anspruch 2 der Formel I, worin R¹ für H oder Phenyl steht, R² für Phenyl, halogen- oder CF₃-substituiertes Phenyl, Thienyl, halogensubstituiertes Thienyl oder Furanyl steht.

4. Verbindungen nach einem der vorhergehenden Ansprüche, wobei R⁸ für H und R⁹ für SO₂ C₁-C₈-Alkyl oder SO₂Phenyl stehen, wobei der Alkylrest der Sulfonylgruppe gegebenenfalls durch ein oder mehrere Halogenatome und der Phenylrest gegebenenfalls durch ein oder mehrere C₁-C₈-Alkylreste substituiert ist.

5. Verbindungen nach Anspruch 4, wobei R⁹ für SO₂CF₃, SO₂CH₃, SO₂Phenyl oder SO₂Tolyl steht.

6. [6-(4-Trifluormethylphenyl)-2,2-dimethyl-7-phenyl-2,3-dihydro-1H-pyrrolizin-5yl]essigsäuremethansulfonimid.

7. Pharmazeutisches Mittel, enthaltend wenigstens eine Verbindung nach einem der Ansprüche 1 bis 6, gegebenenfalls in Kombination mit pharmazeutisch verträglichen Träger- und/oder Zusatzstoffen.

8. Verwendung wenigstens einer Verbindung nach einem der Ansprüche 1 bis 6 zur Herstellung eines pharmazeutischen Mittels zur Prävention von allergisch induzierten Erkrankungen oder zur Behandlung von Erkrankungen des rheumatischen Formenkreises.

9. Verfahren zur Herstellung der Verbindungen nach einem der Ansprüche 1 bis 5, **dadurch gekennzeichnet, daß** man eine Verbindung der allgemeinen Formel II: 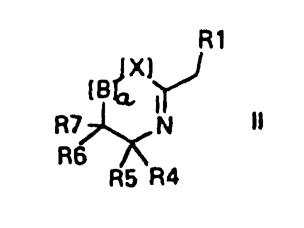 mit einer Verbindung der allgemeinen Formel III: 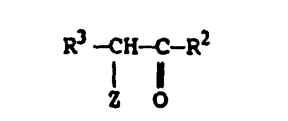 wobei in den obigen Formeln B und a und zwei der Reste R¹, R² und R³ die in Anspruch 1 angegebenen Bedeutungen besitzen und der dritte für ein Wasserstoffatom steht und Z für Cl oder Br steht, zu einer Verbindung der allgemeinen Formel Ia: 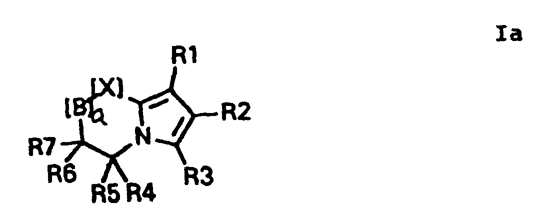 worin R¹ bis R⁷, B, a und X die oben angegebenen Bedeutungen besitzen, umsetzt und
in die erhaltene Verbindung den Rest A-CO₂H einführt und die erhaltene Säure in das gewünschte Sulfonamid überführt.

10. Verfahren nach Anspruch 9, **dadurch gekennzeichnet, daß** man eine Verbindung der Formel Ia, worin einer der Reste R¹, R² und R³ für A-CO₂H steht, in Form eines aktivierten Derivates mit dem entsprechenden Sulfonamid, gegebenenfalls in Gegenwart einer Base, umsetzt.

11. Verfahren zur Herstellung der Verbindungen der Formel I, worin X für O oder S steht, **dadurch gekennzeichnet, daß** man eine Verbindung der Formel VI: 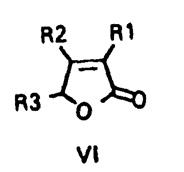 worin R¹, R² und R³ die in Anspruch 1 angegebenen Bedeutungen besitzen, mit einer Verbindung der Formel 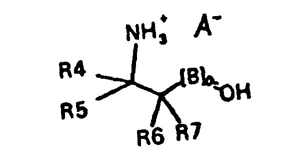 worin A⁻ für ein Anion steht, B, a und R⁴ bis R⁷ die in Anspruch 1 genannten Bedeutungen besitzen, zu einer Verbindung der Formel VII: 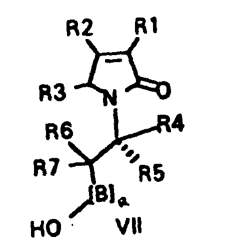 umsetzt und die Verbindung der Formel VII zu einer Verbindung der Formel 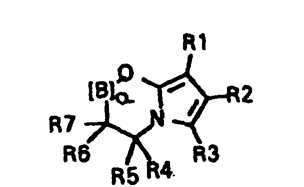 cyclisiert, vorzugsweise mit Hilfe von Polyphosphorsäure, oder eine Verbindung de Formel VII in Anwesenheit von Phosphorpentasulfid zu einer Verbindung der Formel 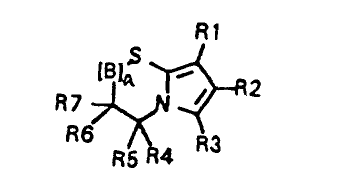 cyclisiert.

12. Verfahren zur Herstellung der Verbindungen der Formel I' 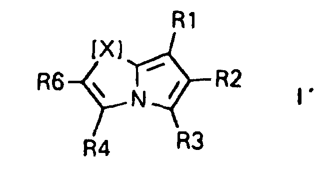 worin R¹, R², R³, R⁴ und R⁶ die in Anspruch 1 angegebenen Bedeutungen besitzen und X für 0 oder S steht, **dadurch gekennzeichnet, daß** man eine Verbindung der in Anspruch 11 definierten Formel VI mit einer Verbindung der Formel 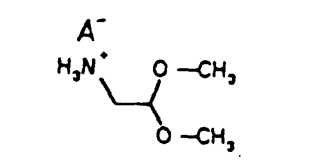 worin A⁻ für ein Anion steht, zu einer Verbindung der Formel VIII 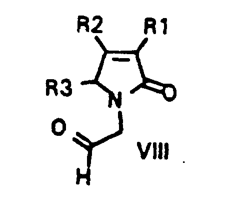 umsetzt und
a) eine Verbindung der Formel VIII zu einer Verbindung der Formel I', worin X für O steht, cyclisiert, vorzugsweise mit Hilfe von Polyphosporsäure (PPA), oder
b) eine Verbindung der Formel VIII mit Phosphorpentasulfid zu einer Verbindung der Formel I', worin X für S steht, cyclisiert.

## Claims

1. Heterocyclic compounds of formula (I) 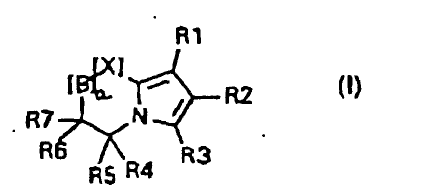 wherein
two of the groups R¹, R² and R³, which can be the same or different, denote a hydrogen atom, a phenyl or naphthyl group, optionally substituted by one or two substituents selected from halogen, pseudohalogen, CF₃, NO₂, OH, C₁-C₈ alkoxy, OCF₃, C₁-C₈ alkyl or phenoxy; or an aromatic heterocyclic group which is selected from thiophenyl, thiazolyl, thiadiazolyl, furanyl, pyridinyl, pyrimidinyl, benzofuranyl or quinolinyl and which is optionally substituted by halogen, CF₃, C₁-C₈ alkyl or C₁-C₈ alkoxy, and
the third of the groups R¹, R² and R³ denotes A-Y
A denotes C₁-C₈ alkylene or C₂-C₈ alkenylene
Y denotes CONR⁸R⁹
R⁸ denotes hydrogen or C₁-C₈ alkyl
R⁹ denotes SO₂-C₁-C₈ alkyl, optionally substituted by halogen
or SO₂ phenyl, optionally substituted by C₁-C₈ alkyl,
R⁴, R⁵, R⁶ and R⁷, which may be the same or different, denote H or C₁-C₈ alkyl or two of the adjacent groups denote a chemical bond between the two ring atoms to which they are bonded, the two other groups having the meanings as given,
X denotes CH₂, O or S;
B denotes CH₂; and
a denotes 0, 1 or 2, and
the optical isomers, pharmaceutically-compatible salts and physiologically readily-hydrolysable esters thereof.

2. Compounds according to claim 1 of formula (I) wherein two of the groups R¹, R² and R³, independently from each other, denote a hydrogen atom, a phenyl group, a phenyl group substituted by 1 or 2 halogen atoms or CF₃ or an optionally-substituted aromatic, heterocyclic group as defined in claim 1,
the third of the groups R¹, R² and R³ denotes A-Y, wherein
A denotes C₁-C₈ alkylene and
Y is as defined in claim 1.

3. Compounds according to claim 2 of formula (I), wherein R¹ denotes H or phenyl, R² denotes phenyl, halogen- or CF₃-substituted phenyl, thienyl, halogen-substituted thienyl or furanyl.

4. Compounds according to one of the preceding claims, wherein R⁸ denotes H and R⁹ denotes SO₂-C₁-C₈ alkyl or SO₂ phenyl, wherein the alkyl group of the sulphonyl group is optionally substituted by one or more halogen atoms and the phenyl group is optionally substituted by one or more C₁-C₈ alkyl groups.

5. Compounds according to claim 4, wherein R⁹ denotes SO₂CF₃, SO₂CH₃, SO₂ phenyl or SO₂ tolyl.

6. [6-(4-trifluoromethylphenyl)-2,2-dimethyl-7-phenyl-2,3-dihydro-lH-pyrrolizin-5-yl]acetic acid methane sulphonimide.

7. Pharmaceutical composition comprising at least one compound according to one of claims 1 to 6, optionally in combination with pharmaceutically-compatible carriers- and/or additives.

8. Use of at least one compound according to one of claims 1 to 6 for the manufacture of a pharmaceutical composition for the prevention of allergically-induced illnesses or for the treatment of rheumatic illnesses.

9. A process for the preparation of compound according to one of claims 1 to 5, **characterised in that** a compound of general formula (II) 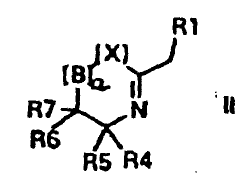 is reacted with a compound of general formula (III) 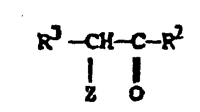 wherein in the above formulae B and a and two of the groups R¹, R² and R³ are as defined in claim 1 and the third denotes a hydrogen atom and Z denotes Cl or Br, to give a compound of general formula Ia: 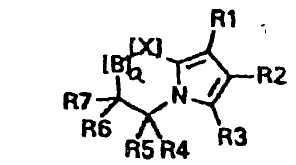 wherein R¹ to R⁷ ,B, a and X are as defined above, and
the group A-CO₂H is inserted into the obtained compound and the obtained acid is converted into the desired sulphonamide.

10. A process according to claim 9, **characterised in that** a compound of formula Ia, wherein the groups R¹, R² and R³ denote A-CO₂H, is reacted in the form of an active derivative with the appropriate sulphonamide, optionally in the presence of a base.

11. A process for the preparation of a compound of formula I wherein X denotes O or S, **characterised in that** a compound of formula VI: 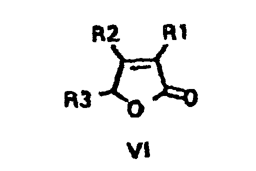 wherein R¹, R² and R³ are as defined in claim 1 is reacted with a compound of formula 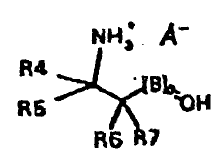 wherein A⁻ denotes an anion, B, a and R⁴ to R⁷ are as defined in claim 1; to give a compound of formula VII: 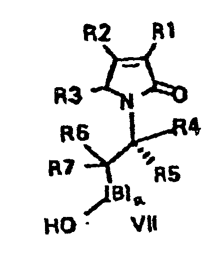 and the compound of formula VII is cyclised to give a compound of formula: 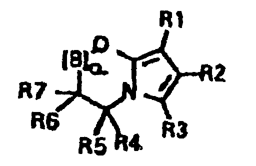 preferably with the help of polyphosphoric acid, or the compound of formula VII is cyclised in the presence of phosphorus pentasulphide to give a compound of formula

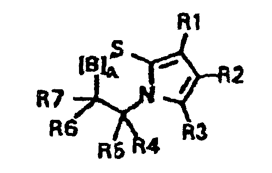

12. A process for the preparation of compounds of formula I' 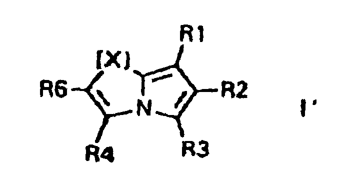 wherein R¹, R², R³, R⁴ and R⁶ are as defined in claim 1 and X denotes O or S, **characterised in that** a compound of formula (VI), as defined in claim 11, is reacted with a compound of formula 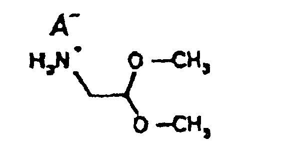 wherein A⁻ denotes an anion, to give a compound of formula (VIII) 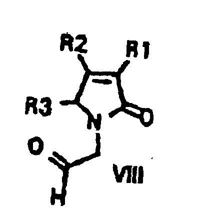 and
a) a compound of formula VIII is cyclised to give a compound of formula I', wherein X denotes O, preferably with the help of polyphosphoric acid (PPA) ; or
b) a compound of formula VIII is cyclised with phosphorus pentasulphide to give a compound of formula I⁻, wherein X denotes S.

## Revendications

1. Composés hétérocycliques de formule (I) : 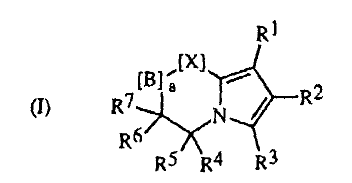 dans laquelle
deux des résidus R¹, R² et R³, identiques ou différents, représentent un atome d'hydrogène, un résidu phényle ou naphtyle portant éventuellement un ou deux substituants choisis parmi les atomes d'halogène ou pseudohalogène, CF₃, NO₂, OH, alcoxy en C₁₋₈, OCF₃, alkyle en C₁₋₈ ou phénoxy, ou un résidu hétérocyclique aromatique choisi parmi les groupes thiophène, thiazole, thiadiazole, furane, pyridine, pyrimidinc, benzofurane ou quinoléine, et portant éventuellement des substituants halogéno, CF₃, alkyle en C₁₋₈ ou alcoxy en C₁₋₈, et le troisième des résidus R¹, R² et R³ représente un groupe A-Y,
A représente un groupe alkylène en C₁₋₈ ou alcénylène en C₂₋₈,
Y représente un groupe CONR⁸R⁹,
R⁸ représente un atomc d'hydrogène ou un groupe alkyle en C₁₋₈,
R⁹ représente un groupc SO₂-alkyle en C₁₋₈, portant éventuellement des substituants halogéno, ou un groupe SO₂-pbényle portant éventuellement des substituants alkyle en C₁₋₈,
R⁴, R⁵, R⁶ et R⁷, identiques ou différents, représentent chacun un atomc d'hydrogène ou un groupe alkyle en C₁₋₈, ou deux résidus vicinaux représentent unc liaison chimique entre les deux atomes du cycle auxquel ils sont liés, ct les autres ont la signification indiquée,
X représente un groupe CH₂, O ou S,
B représente un groupe CH₂, et
a vaut 0, 1 ou 2, et
les isomères optiques, sels parmaceutiquement acceptables et esters facilement hydrolysables de tels composés.

2. Composés selon la revendication 1 de formule (I) dans lesquels deux des résidus R¹. R² et R³ représentent, indépendamment l'un de l'autre, un atome d'hydrogène, un résidu phényle, un résidu phényle portant 1 ou 2 substituants halogéno ou CF₃, ou un résidu hélérocyclique aromatique éventuellement substitué tel que défini dans la revendication 1, le troisième des résidus R¹, R² et R³ représente un groupc A-Y, où A représente un groupe alkylène en C₁₋₈ ct Y a la signification indiquée dans la revendication 1.

3. Composés selon la revendication 2 de formule (I) dans lesquels R¹ représente un atome d'hydrogène ou un groupe phényle, R² représente un groupe phényle, un groupe phényle halogéné ou trifluorométhylé, thiényle, thiényle halogéné ou furanyle.

4. Composés selon l'une quelconque des revendications précédentes, dans lesquels R⁸ représente un atome d'hydrogène et R⁹ un groupe SO₂-(alkyle en C₁₋₈) ou SO₂-phényle, le résidu alkyle porté par le groupe sulfonyle portant éventuellement un ou plusieurs atomes d'halogène et le résidu phényle portant éventuellement un ou plusieurs substituants alkyle en C₁₋₈.

5. Composés selon la revendication 4, dans lesquels R⁹ représente un résidu SO₂CF₃, SO₂CH₃, SO₂-phényle ou SO₂-toluyle.

6. Imide d'acide [6-(4-trifluorométhylphényl)-2,2-diméthyl-7-phényl-2,3-dihydro-1H-pyrrolizin-5-yl)]-acétique et d'acide méthanesulfonique.

7. Composition pharmaceutique contenant au moins un composé selon une des revendications 1 à 6, éventuellement en combinaison avec un excipient et/ou des additifs pharmacologiquement acceptables.

8. Utilisation d'au moins un composé selon une des revendications 1 à 6, pour la fabrication d'une composition pharmaceutique destinée à la prévention de maladies d'origine allergique ou pour le traitement de maladies rhumatismales,

9. Procédé de préparation de composés selon l'une quelconque des revendications 1 à 5, **caractérisé par le fait que** l'on fait réagir un composé de formule générale (II) : 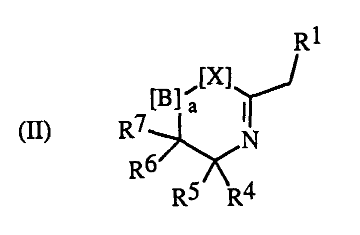 avec un composé de formule générale (III) 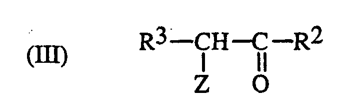 les symboles B et a et deux des résidus R¹, R² et R³, dans les formules ci-dessus, ayant la signification indiquée dans la revendication 1, et le troisième représentant un atome d'hydrogène, et Z représente un atome de chlore ou de brome, de manière à obtenir un composé de formule générale (Ia) 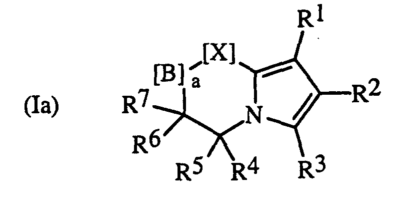 dans laquelle R¹ à R⁷, B, a, et X ont la signification indiquée ci-dessus, et que l'on introduit dans le composé obtenu le résidu A-CO₂H et que l'on convertit l'acide obtenu en sulfonamide.

10. Procédé selon la revendication 9, **caractérisé par le fait que** l'on fait réagir un composé de formule (Ia) dans lequel un des résidus R¹, R² et R³ représente un résidu A-CO₂H, sous forme d'un dérivé activé avec le sulfonamide approprié, éventuellement en présence d'une base.

11. Procédé de préparation de composés de formule (I) dans laquelle X représente un atome d'oxygène ou un atome de soufre, **caractérisé par le fait que** l'on fait réagir un composé de formule (VI) 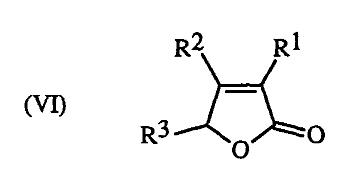 dans laquelle R¹ R² et R³ ont la signification indiquée dans la revendication 1, avec un composé de formule 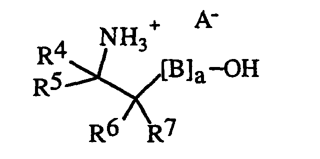 dans laquelle A- représente un anion, B, a et R⁴ à R⁷ ont la signification indiquée dans la revendication 1, de manière à obtenir un composé de formule (VII) 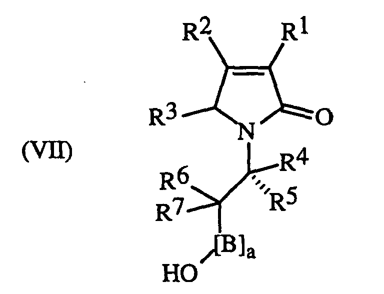 et que l'on procède ensuite à une cyclisation du composé de formule (VII) pour obtenir un composé de formule 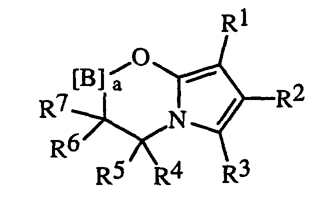 de préférence en présence d'acide polyphosphorique, ou que l'on procède à la cyclisation d'un composé de formule (VII) en présence de pentasulfure de phosphore pour obtenir un composé de formule

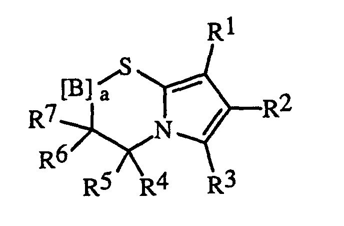

12. Procédé de préparation de composés de formule (I') 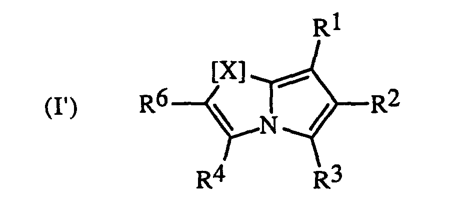 où R¹, R², R³, R⁴ et R⁶ ont les significations indiquées dans la revendication 1, et où X représente un atome d'oxygène ou de soufre, **caractérisé par le fait que** l'on fait réagir un composé de formule (VI) tel que défini dans la revendication 11, avec un composé de formule 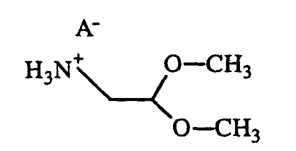 où A⁻ représente un anion, de manière à obtenir un composé de formule (VIII), et que l'on procède ensuite à une cyclisation d'un composé de formule (VIII) 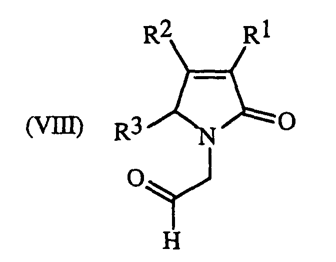
a) de préférence en présence d'acide polyphosphorique, de manière à obtenir un composé de formule (I') où X représente un atome d'oxygène, ou
b) en présence de pentasulfure de phosphore, de manière à obtenir un composé de formule (I') où X représente un atome de soufre.
